# EUROPEAN PATENT SPECIFICATION

(11) **EP 0 443 324 B1**
(45) Date of publication and mention of the grant of the patent: **08.11.1995**
(21) Application number: 91100559.3
(22) Date of filing: 18.01.1991
(51) Int. Cl.: B01F 5/10, A61M 1/16

(54) **System for preparing a concentrate solution**
System zur Herstellung einer konzentrierten Lösung
Système pour la peparation d'un fluide concentré

(30) Priority: 19.02.1990 SE 9000586
(43) Date of publication of application: 28.08.1991
(60) Divisional application: 94101963.0
(73) Proprietor: GAMBRO AB, S-220 10 Lund (SE)
(72) Inventor: Jönsson, Lennart, S-240 20 Furulund (SE); Knutsson, Stefan, S-237 00 Bjärred (SE)
(74) Representative: Asketorp, Göran

(56) References cited:
- EP-A- 0 278 100
- DE-A- 2 924 406
- FR-A- 1 333 222
- US-A- 4 082 667
- US-A- 4 848 916

## Description

The present invention relates to a system for preparing a concentration solution for use as a medical solution, for example, dialysis fluid or replacement fluid for hemofiltration or a concentrate for preparation of such fluids.

Previous systems available for similar purposes normally start from one or more concentrates in liquid form.

US-A-4 158 034 discloses how a concentrate in liquid form is mixed with water for preparation of a dialysis fluid. EP-A-0 022 922 and US-A-4 783 273 disclose how two liquid-based concentrates are mixed with water to obtain a fluid intended for medical use, for example dialysis fluid. More recently, systems have been introduced using one or more concentrates in powder form. US-A-4 784 495 discloses how one or more such powder-based concentrates can be used, possibly with the addition of a liquid-based concentrate.

US-A-4 848 916 discloses an apparatus for bulk mixing of bicarbonate solutions by adding bicarbonate powder to water enclosed in a mixing vessel. In order to promote the dissolution of the powder, the water is recirculated in an external recirculation circuit until the powder is completely dissolved.

If a concentrate in powder form is used, where the powder is hard to dissolve, it can be difficult to achieve an adequately fast dissolving in the system according to US-A-4 784 495. Furthermore, the system according to said patent can become relatively complicated if it is desired to add several different, individually pre-packed powder-based concentrates.

### DISCLOSURE OF THE INVENTION

The above-mentioned problems are solved by the present invention which relates to a system for preparing a concentrate solution for use as a medical solution, for example, dialysis fluid or replacement fluid or hemofiltration or a concentrate fluid for preparation of such fluids, comprising a container, a supply means of pure water for supplying a predetermined amount of water to said container, recirculation means for recirculating said predetermined amount of water in a recirculation path including said container. According to the invention, the system comprises a plurality of vessels, each containing an in water dissolvable solid substance, said recirculation means recirculating said predetermined amount of water in said recirculation path including said container and a first of said vessels, for at least partially dissolving the substance of said first vessel until a predetermined concentration is obtained for providing a partially prepared solution, said recirculation means further comprising valve means for connecting said container to a further of said plurality of vessels for including each vessel one by one in the recirculation path replacing the previous vessel, for recirculating said partially prepared solution in said recirculation path for at least partially dissolving the substance of each of said vessels until a predetermined concentration is obtained for providing said concentrate solution.

By means of this recirculation even a relatively difficult to dissolve powder-based concentrate can be dissolved. At the same time a liquid-based concentrate can be prepared in the container from one or more powder-based concentrates which, after preparation, can be conveyed to conventional dialysis machines which otherwise normally receive such liquid-based concentrates from one or more external containers.

Preferably, the system according to the invention comprises determination means included within the recirculation path for determining the concentration level of the partially prepared solution of said recirculation path, whereby said recirculation means further comprising control means for terminating the flow through the one of said vessels, which is connected to the recirculation path, when a predetermined concentration level has been obtained as determined by said determination means.

Thus, precisely the right amount of concentrate for the patient can be taken from each vessel. In other words an individual dosage can be obtained.

Such determination means can be a conductivity meter or other suitable means for measuring the concentration, such as a pH-meter or an ion-selective meter. The same substitution can also be made for the conductivity meters mentioned in the rest of this description.

Further details and features are included in the subclaims. Further advantageous objects and features appear from the following detailed description of a preferred embodiment with reference to the drawings.

Figures 1-3 show in the form of a block diagram a preferred embodiment of the system according to the invention in its three different connectable positions.

Fig. 4 shows a diagram of the measured conductivity in the above-described recirculation circuit with the supply of three different concentrates.

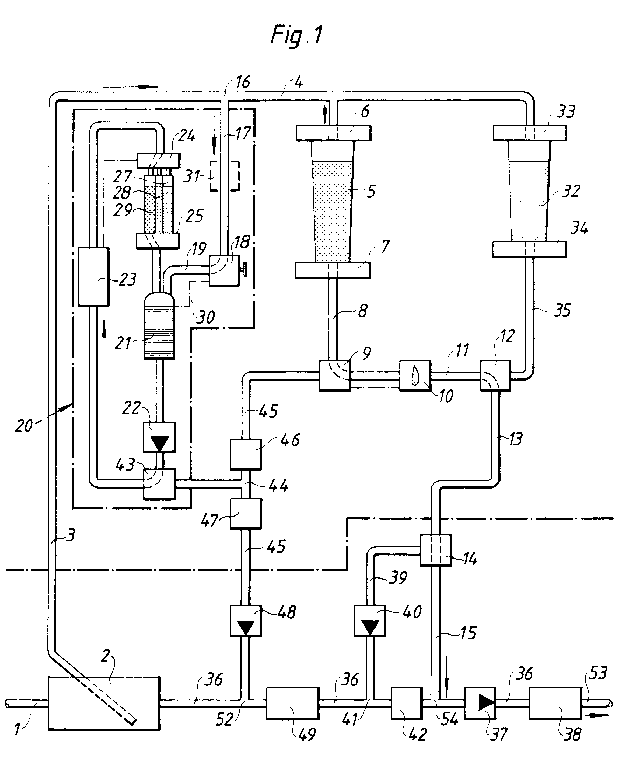

Figures 1-3 are block diagrams of a preferred embodiment of the system according to the invention in its three different positions. Figure 1 shows how a recirculation circuit 20 and a separate powder cartridge 5 are filled with water.

The water is taken from an inlet 1 via a heating vessel 2 through conduits 3 and 4 to the powder cartridge 5, which is located between two connection pieces 6 and 7. When the cartridge 5 is filled, the water is further passed as shown in figure 1 through a conduit 8 and a valve 9 to a detector 10, which detects the presence of water and/or concentrate therein. The detection can include a certain time delay so that the fluid will have time to fill the subsequent conduit 11, valve 12, conduit 13, valve 14 and conduit 15.

At the same time, water is conveyed from a branch point 16 through a conduit 17, a valve 18 and a conduit 19 to a recirculation circuit, which in its entirety is denoted by 20.

This circuit includes a mixing vessel 21, a recirculation pump 22, a concentration measuring device 23, for example a conductivity meter, and connected between two valve units 24 and 25 a number of cartridges or other vessels 27, 28 and 29 for one or more powder concentrates and possibly some liquid-based concentrate. When a sufficient quantity fluid is supplied to the mixing vessel 21, the valve 18 is closed, which can occur under the control of a level indicator schematically shown by dashed line 30.

Then, the recirculation pump 22 is started with the valve units 24 and 25 so positioned that the water passes through one of the vessels or cartridges 27-29. In figure 1, vessel 29 is connected. When the desired conductivity is obtained, which is monitored by help of the meter 23, the next cartridge 28 is connected, and then finally cartridge 27.

If the prepared fluid in the mixing vessel 21 should include another liquid-based or easily dissolvable concentrate, the dissolving can occur during the filling of the mixing vessel 21 with the vessel containing this concentrate being connected to the conduit 17. Such a vessel 31 is indicated in block-form in dashed lines.

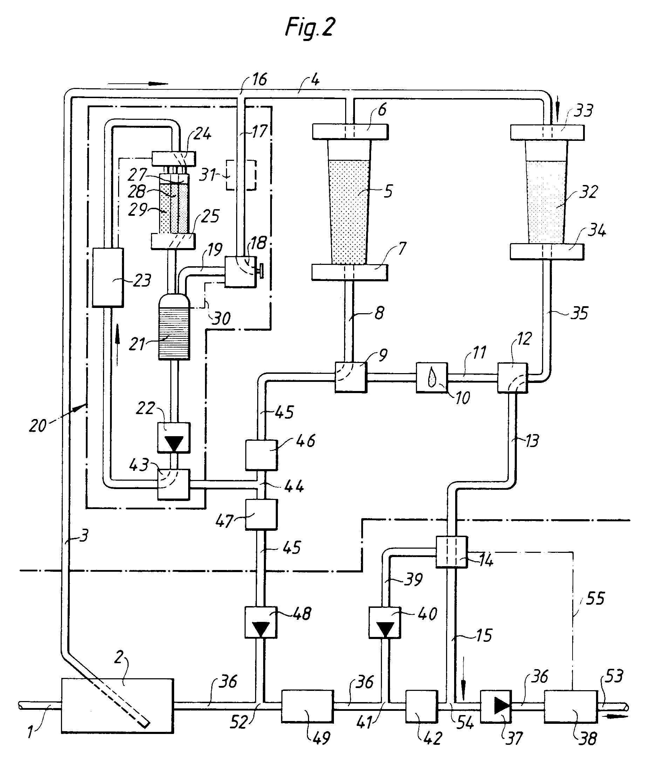

When the recirculation circuit 20 is filled, the valve 18 is closed as showed in figure 2.

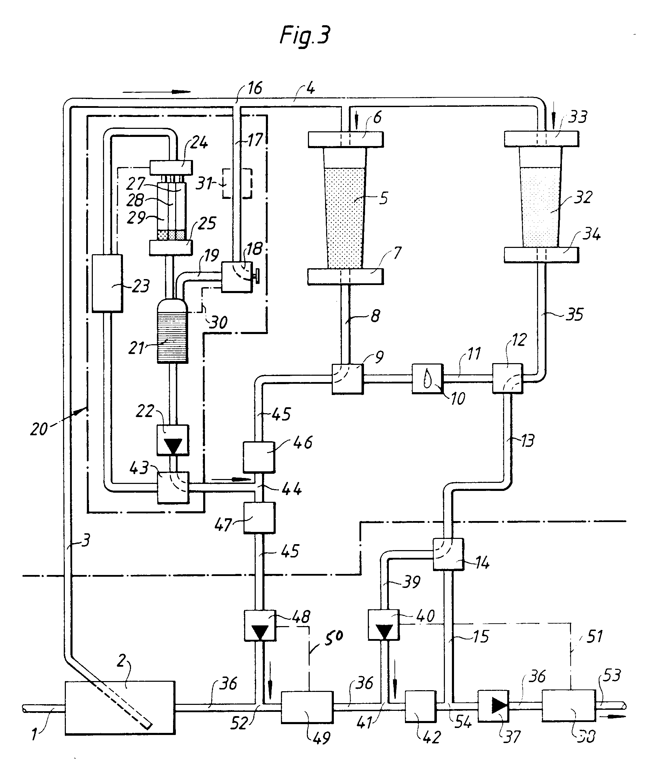

Similarly, valve 9 is actuated when vessel 5 is filled. This position is also shown in figure 2. Simultaneously, valve 12 is switched from the position shown in figure 1 to that in figure 2, so that water from the conduit 4 can pass to a further vessel or powder cartridge 32 located between two connection pieces 33 and 34. When this cartridge 32 has been filled, the water with dissolved concentrate therein is conveyed through the conduit 35, valve 12, conduit 13, valve 14 and conduit 15 to a main line or conduit 36 which includes a pump 37 and a detector 38, for example a conductivity measuring device. This detector controls the valve 14 so that it is switched to the position shown in figure 3. The fluid from vessel 32 is now conveyed instead from valve 14 via a conduit 39 with a dosage pump 40 to the main line 36 to a mixing point 41 upstream of a restrictor 42. This restrictor 42, together with the pump 37 and a gastrap not shown arranged further downstream, are used for de-gasing the prepared fluid.

When valve 14 has been switched to the position shown in figure 3, the described system is ready for dialysis. An inlet valve 43 in the recirculation circuit 20 is thus actuated so the pump 22 can pump prepared fluid from the mixing vessel 21 to a mixing point 44, to which concentrate from vessel 5 is conveyed via valve 9 and conduit 45. The appropriate concentration of the dissolved substance is measured in this conduit upstream and downstream of the mixing point 44. This can be achieved, for example, by means of a differential conductivity meter, whose measuring points are denoted by 46 and 47, respectively. The prepared solution can then be conveyed further through the conduit 45 with the help of a pump 48, which preferably consists of an accurately metering dosage pump, and finally to the main line 36, in which the concentration is once more checked by means of a meter 49, for example another conductivity measuring device. This meter 49 then controls the pump 48, as shown by dashed line 50 in figure 3. Additional concentrate is then added to the prepared solution at the mixing point 41 by means of a pump 40 controlled by the measuring device 38 as indicated by the dashed line 51. By means of this measuring device 38, which preferably consists of a conductivity meter, the final concentration of the prepared solution obtained from the system according to the invention is regulated.

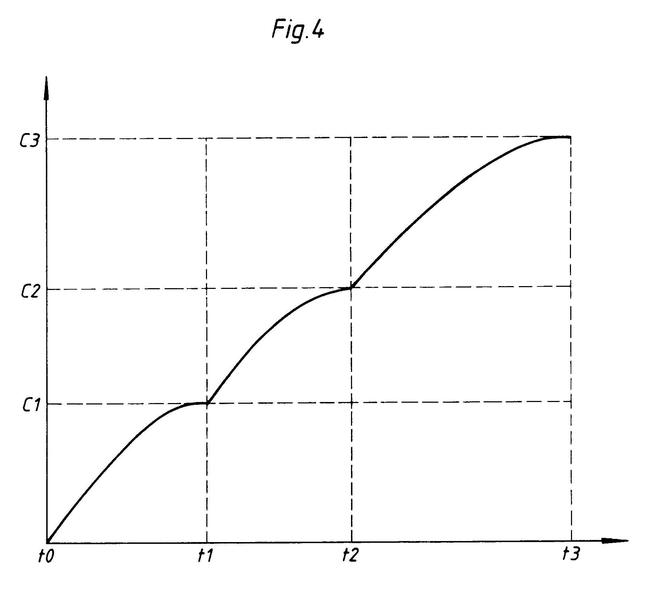

Finally, figure 4 shows a diagram of how the conductivity in the recirculation circuit 20 varies. Here it is assumed that the meter 23 consists of a conductivity measuring device and that the vessels 27, 28 and 29 contain three different salts. Thus, between time t0 and t1 one of these vessels is connected until the conductivity value c1 is obtained. Thereafter, the next vessel is connected until the conductivity value c2 is reached. Then the last vessel is connected which remains connected until the conductivity obtains the desired value c3. At this point the valve 43 is switched over so that the ready-prepared solution can be conducted to the mixing point 44. At this mixing point concentrate from the vessel 5 is added. The mixture thus obtained is then led to a mixing point 52 in the main line 36 where it is mixed with water from the heating vessel 2. At the next mixing point 41, concentrate from the vessel 32 is then added. After a final check in the measuring device 38, the ready prepared solution can then be conducted to its place of consumption, as symbolized by an arrow 53.

## Claims

1. System for preparing a concentrate solution for use as a medical solution, for example, dialysis fluid or replacement fluid for hemofiltration or a concentrate fluid for preparation of such fluids, comprising
a container (21);
water supply means (17) for supplying a predetermined amount of water to said container;
recirculation means (22) for recirculating said predetermined amount of water in a recirculation path including said container (21);
**characterized** by
a plurality of vessels (27, 28, 29), each containing an in water dissolvable solid substance;
said recirculation means (22) recirculating said predetermined amount of water in said recirculation path including said container (21) and a first of said vessels (29), for at least partially dissolving the substance of said first vessel until a predetermined concentration is obtained for providing a partially prepared solution;
said recirculation means further comprising valve means (24, 25) for connecting said container (21) to a further of said plurality of vessels (27,28) for including each vessel one by one in the recirculation path replacing the previous vessel, for recirculating said partially prepared solution in said recirculation path for at least partially dissolving the substance of each of said vessels until a predetermined concentration is obtained for providing said concentrate solution.

2. System according to claim 1, **characterized** by
determination means (23) included within the recirculation path for determining the concentration level of the partially prepared solution of said recirculation path;
said recirculation means further comprising control means (22, 43), for terminating the flow through the one of said vessels, which is connected to the recirculation path, when a predetermined concentration level has been obtained as determined by said determination means.

3. System according to claim 2, **characterized** in that said determination means comprises a conductivity meter (23) for determining the conductivity and thus the concentration level of said partially prepared solution.

4. System according to any one of the previous claims, **characterized** in that said recirculation means comprises pump means (22) for recirculating the partially prepared solution in the recirculation path.

5. System according to any one of the previous claims, **characterized** by withdrawal means (43) for withdrawing prepared concentrate solution from said container.

6. System according to any one of the previous claims, **characterized** in that said recirculation means comprises a pump means (22) for metering the concentrate solution to an outlet conduit for using said concentrate solution as a medical solution.

7. System according to claim 6, **characterized** in that said recirculation means comprises an outlet valve (43) for connecting said container to said outlet conduit.

8. System according to any one of the previous claims, **characterized** by a heating device (2) for heating said supplied water.

9. System according to any one of the previous claims, **characterized** in that said supply means for water comprises a valve means (18) for terminating said supply of water when a predetermined amount of water has been supplied, as determined by for example a level indicator (30).

10. System according to any one of the previous claims, **characterized** in that said plurality of vessels contains an excess of solid substances, so that said concentrate solution can be prepared with the content of said plurality of vessels.

11. System according to any one of the previous claims, **characterized** in that there are three vessels (27, 28, 29) containing three different salts.

12. System according to any one of the previous claims, **characterized** in that each of said plurality of vessels is a cartridge.

13. System according to anyone of the previous claims, **characterized** by a further vessel (31) connected in parallel with said plurality of vessels and containing a liquid-based concentrate solution intended to be included in the concentrate solution.

14. System according to claim 13, **characterized** in that said further vessel (31) is connected to said water supply means (17) for mixing said liquid-based concentrate solution with water and supplying said mixture to said container (21).

15. System according to any one of the previous claims, **characterized** by a level indicator (30) for terminating the supply of water when a predetermined amount of water has been supplied.

16. System according to any one of the previous claims, **characterized** in that said recirculation means comprises a recirculation conduit for forming said recirculation path, comprising said container (21), said valve means (24, 25) and one of said vessels (29).

17. System according to any one of the previous claims, **characterized** by further comprising
a secondary vessel (5), containing an in water dissolvable solid substance;
secondary water supply means (4) for supplying water to said secondary vessel for dissolving said solid substance and forming a secondary concentrate solution;
mixing means (44) for mixing said secondary concentrate solution with said concentrate solution withdrawn from said recirculation means for forming said medical solution.

18. System according to claim 17, **characterized** by further comprising
a tertiary vessel (32), containing an in water dissolvable solid substance;
tertiary water supply means (4) for supplying water to said tertiary vessel for dissolving said solid substance and forming a tertiary concentrate solution;
mixing means (41) for mixing said tertiary concentrate solution with said concentrate solution withdrawn from said recirculation means, possibly mixed with said secondary concentrate solution for forming said medical solution.

19. System according to any one of the previous claims, **characterized** by a conduit (3, 17) for supplying water from said water supply means (2) straight to one of said vessels (31, 27, 28, 29) for flushing therefrom a predetermined quantity of said solid substance or liquid-based concentrate solution directly to the container (21).

## Patentansprüche

1. System zur Herstellung einer konzentrierten Lösung für die Verwendung als eine medizinische Lösung, wie beispielsweise eine Dialyseflüssigkeit oder Austauschflüssigkeit für Hämofiltration oder eine Konzentratflüssigkeit zur Herstellung solcher Flüssigkeiten, mit einem Behältnis (21), Wasserzufuhreinrichtungen (17) für die Zufuhr einer bestimmten Wassermenge zu dem Behältnis und eine Rezirkuliereinrichtung (22) zum Rezirkulieren der vorbestimmten Wassermenge auf einem Rezirkulierweg einschließlich des Behältnisses (21), **gekennzeichnet durch** mehrere Behälter (27, 28, 29), von denen jeder eine in Wasser lösbare feste Substanz enthält,
wobei die Rezirkuliereinrichtung (22) die vorbestimmte Wassermenge auf dem Rezirkulierweg einschließlich des Behältnisses (21) und eines ersten der Behälter (29) rezirkuliert, um die Substanz des ersten Behälters wenigstens teilweise aufzulösen, bis eine vorbestimmte Konzentration erhalten wird, um eine zum Teil bereitete Lösung zu liefern, und
die Rezirkuliereinrichtung weiterhin Ventileinrichtungen (24, 25) zur Verbindung des Behältnisses (21) mit einem weiteren der mehreren Behälter (27, 28) für einen Einschluß eines jeden Behälters einen nach dem anderen in den Rezirkulierweg unter Ersatz des vorausgehenden Behälters umfaßt, um die zum Teil bereitete Lösung auf dem Rezirkulierweg zu rezirkulieren und so die Substanz in jedem der Behälter wenigstens teilweise aufzulösen, bis eine vorbestimmte Konzentration erhalten wird, um die konzentrierte Lösung zu ergeben.

2. System nach Anspruch 1, **gekennzeichnet durch** in den Rezirkulierweg eingeschlossene Bestimmungseinrichtungen (23) zum Bestimmen der Konzentration der zum Teil bereiteten Lösung des Rezirkulierweges, wobei die Rezirkuliereinrichtung weiterhin Steuereinrichtungen (22, 43) zur Beendigung des Flusses durch einen der Behälter, welcher mit dem Rezirkulierweg verbunden ist, wenn eine vorbestimmte Konzentration erreicht wurde, wie durch die Bestimmungseinrichtung ermittelt wird, umfaßt.

3. System nach Anspruch 2, **dadurch gekennzeichnet**, daß die Bestimmungseinrichtung ein Leitfähigkeitsmeßgerät (23) zur Bestimmung der Leitfähigkeit und so der Konzentration der zum Teil bereiteten Lösung umfaßt.

4. System nach einem der vorausgehenden Ansprüche, **dadurch gekennzeichnet**, daß die Rezirkuliereinrichtung eine Pumpeinrichtung (22) zum Rezirkulieren der zum Teil bereiteten Lösung in dem Rezirkulierweg umfaßt.

5. System nach einem der vorausgehenden Ansprüche, **gekennzeichnet durch** Entnahmeeinrichtungen (43) zur Entnahme von bereiteter konzentrierter Lösung aus dem Behältnis.

6. System nach einem der vorausgehenden Ansprüche, **dadurch gekennzeichnet**, daß die Rezirkuliereinrichtung eine Pumpeinrichtung (22) zum Zudosieren der konzentrierten Lösung zu einer Auslaßleitung für eine Verwendung dieser konzentrierten Lösung als eine medizinische Lösung umfaßt.

7. System nach Anspruch 6, **dadurch gekennzeichnet**, daß die Rezirkuliereinrichtung ein Auslaßventil (43) zum Verbinden des Behälters mit der Auslaßleitung umfaßt.

8. System nach einem der vorausgehenden Ansprüche, **gekennzeichnet durch** eine Heizeinrichtung (2) zum Erhitzen des zugeführten Wassers.

9. System nach einem der vorausgehenden Ansprüche, **dadurch gekennzeichnet**, daß die Zufuhreinrichtung für Wasser eine Ventileinrichtung (18) zur Beendigung der Wasserzufuhr, wenn eine vorbestimmte Wassermenge zugeführt wurde, wie beispielsweise durch einen Füllstandsanzeiger (30) bestimmt wird, umfaßt.

10. System nach einem der vorausgehenden Ansprüche, **dadurch gekennzeichnet**, daß die mehreren Behälter einen Überschuß an festen Substanzen enthalten, so daß die konzentrierte Lösung mit dem Gehalt dieser mehreren Behälter bereitet werden kann.

11. System nach einem der vorausgehenden Ansprüche, **dadurch gekennzeichnet**, daß es drei Behälter (27, 28, 29) gibt, die drei unterschiedliche Salze enthalten.

12. System nach einem der vorausgehenden Ansprüche, **dadurch gekennzeichnet**, daß jeder der mehreren Behälter eine Patrone ist.

13. System nach einem der vorausgehenden Ansprüche, **gekennzeichnet durch** einen weiteren Behälter (31), der parallel zu den mehreren Behältern verbunden ist und eine Konzentratlösung auf Flüssigkeitsbasis enthält, die dazu bestimmt ist, in die Konzentratlösung eingearbeitet zu werden.

14. System nach Anspruch 13, **dadurch gekennzeichnet**, daß der weitere Behälter (31) mit der Wasserzufuhreinrichtung (17) zum Vermischen der Konzentratlösung auf Flüssigkeitsbasis mit Wasser und zur Zufuhr dieses Gemisches zu dem Behältnis (21) verbunden ist.

15. System nach einem der vorausgehenden Ansprüche, **gekennzeichnet durch** einen Füllstandanzeiger (30) zur Beendigung der Wasserzufuhr, wenn eine bestimmte Wassermenge zugeführt wurde.

16. System nach einem der vorausgehenden Ansprüche, **dadurch gekennzeichnet**, daß die Rezirkuliereinrichtung eine Rezirkulierleitung zur Bildung des Rezirkulierweges umfaßt, welcher das Behältnis (21), die Ventileinrichtungen (24, 25) und einen der Behälter (29) umfaßt.

17. System nach einem der vorausgehenden Ansprüche, weiterhin **gekennzeichnet durch** einen Sekundärbehälter (5), der eine in Wasser auflösbare feste Substanz enthält,
eine Sekundärwasserzufuhreinrichtung (4) zum Zuführen von Wasser zu dem Sekundärbehälter, um die feste Substanz aufzulösen und eine Sekundärkonzentratlösung zu bilden, und
Mischeinrichtungen (44) zum Mischen der Sekundärkonzentratlösung mit der aus der Rezirkuliereinrichtung entnommenen Konzentratlösung zur Bildung der medizinischen Lösung.

18. System nach Anspruch 17, weiterhin **gekennzeichnet durch**
einen Tertiärbehälter (32) der eine in Wasser auflösbare feste Substanz enthält, eine Tertiärwasserzufuhreinrichtung (4) für die Zufuhr von Wasser zu dem Tertiärbehälter, um die feste Substanz aufzulösen und eine Tertiärkonzentratlösung zu bilden, und
Mischeinrichtungen (41) zum Vermischen der Tertiärkonzentratlösung mit der aus der Rezirkuliereinrichtung abgezogenen Konzentratlösung, gegebenenfalls vermischt mit der Sekundärkonzentratlösung, um die medizinische Lösung zu bilden.

19. System nach einem der vorausgehenden Ansprüche, **gekennzeichnet durch** eine Leitung (3, 17) zur Wasserzufuhr aus der Wasserzufuhreinrichtung (2) gerade zu einem der Behälter (31,27, 28,29) zum Ausschwemmen einer vorbestimmten Menge der festen Substanz oder Konzentratlösung auf Flüssigkeitsbasis daraus direkt zu dem Behältnis (21).

## Revendications

1. Système permettant de préparer une solution de concentré destinée à être utilisée en tant que solution médicale, par exemple fluide de dialyse ou fluide de remplacement pour hémofiltration ou fluide concentré pour la préparation de tels fluides, comprenant :
un réservoir (21),
des moyens de fourniture d'eau (17), servant à fournir une quantité préfixée d'eau au réservoir,
des moyens de recyclage (22), servant a recycler là quantité préfixée d'eau suivant un chemin de recyclage contenant le réservoir (21),
caractérisé par :
plusieurs récipients (27, 28, 29), contenant chacun une substance solide soluble dans l'eau,
les moyens de recyclage (22) recyclant la quantité préfixée d'eau suivant le ledit chemin de recyclage contenant le réservoir (21) et un premier (29) desdits récipients, de façon à dissoudre au moins partiellement la substance du premier récipient, jusqu'à ce qu'une concentration préfixée soit obtenue de façon à fournir une solution partiellement préparée,
les moyens de recyclage comprenant en outre des moyens à fonction de valve (24, 25) servant à faire communiquer le réservoir (21) avec un autre récipient (27, 28) parmi les multiples récipients, de façon à introduire un à un chaque récipient dans le chemin de recyclage en remplacement du récipient précédent, en vue de recycler suivant le chemin de recyclage la solution partiellement préparée, de manière à dissoudre au moins partiellement la substance de chacun des récipients, jusqu'à ce qu'une concentration préfixée soit obtenue de façon à fournir ladite solution de concentré.

2. Système suivant la revendication 1, caractérisé par :
des moyens de détermination (23), interposés dans le chemin de recyclage et servant à déterminer le niveau de concentration de la solution partiellement préparée correspondant à ce chemin de recyclage,
les moyens de recyclage comprenant en outre des moyens de commande (22, 43) servant à interrompre l'écoulement traversant celui des récipients qui est branché sur le chemin de recyclage, lorsqu'a été obtenu un niveau préfixé de concentration déterminé à l'aide des moyens de détermination.

3. Système suivant la revendication 2, caractérisé en ce que les moyens de détermination comprennent un appareil de mesure de conductivité (23) servant à déterminer la conductivité et donc le niveau de concentration de ladite solution partiellement préparée.

4. Système suivant l'une quelconque des revendications précédentes, caractérisé en ce que les moyens de recyclage comprennent des moyens de pompage (22) servant à recycler suivant le chemin de recyclage la solution partiellement préparée.

5. Système suivant l'une quelconque des revendications précédentes, caractérisé par des moyens d'extraction (43) servant à extraire du réservoir la solution de concentré préparée.

6. Système suivant l'une quelconque des revendications précédentes, caractérisé en ce que les moyens de recyclage comprennent des moyens de pompage (22) servant à fournir d'une manière dosée la solution de concentré à une tuyauterie de sortie permettant d'utiliser la solution de concentré en tant que solution médicale.

7. Système suivant la revendication 6, caractérisé en ce que les moyens de recyclage comprennent une valve de sortie (43) servant à faire communiquer le récipient avec la tuyauterie de sortie.

8. Système suivant l'une quelconque des revendications précédentes, caractérisé par un dispositif de chauffage (2) servant à chauffer l'eau fournie.

9. Système suivant l'une quelconque des revendications précédentes, caractérisé en ce que les moyens de fourniture d'eau comprennent des moyens à fonction de valve (18) qui servent à interrompre la fourniture d'eau lorsqu'a été fournie une quantité préfixée d'eau, déterminée par exemple au moyen d'un indicateur de niveau (30).

10. Système suivant l'une quelconque des revendications précédentes, caractérisé en ce que les multiples récipients contiennent un excès de substance solide, de sorte que la solution de concentré peut être préparée avec le contenu des multiples récipients.

11. Système suivant l'une quelconque des revendications précédentes, caractérisé en ce qu'il est prévu trois récipients (27, 28, 29) contenant trois sels différents.

12. Système suivant l'une quelconque des revendications précédentes, caractérisé en ce que chacun des multiples récipients est une cartouche.

13. Système suivant l'une quelconque des revendications précédentes, caractérisé par un récipient supplémentaire (31) branché en parallèle aux multiples récipients et contenant une solution de concentré à base de liquide destinée à faire partie de la solution de concentré.

14. Système suivant la revendication 13, caractérisé en ce que le récipient supplémentaire (31) est mis en communication avec les moyens de fourniture d'eau (17) afin de mélanger la solution de concentré à base de liquide à de l'eau et à fournir ce mélange au réservoir (21).

15. Système suivant l'une quelconque des revendications précédentes, caractérisé par un indicateur de niveau (30) servant à interrompre la fourniture d'eau lorsqu'une quantité préfixée d'eau a été fournie.

16. Système suivant l'une quelconque des revendications précédentes, caractérisé en ce que les moyens de recyclage comprennent une tuyauterie de recyclage qui sert à former le chemin de recyclage et qui comprend le réservoir (21), les moyens à fonction de valve (24, 25) et l'un desdits récipients (29).

17. Système suivant l'une quelconque des revendications précédentes, caractérisé en ce qu'il comprend en outre :
un récipient secondaire (5) contenant une substance solide soluble dans l'eau,
des moyens secondaires de fourniture d'eau (4) servant à fournir de l'eau au récipient secondaire afin de dissoudre la substance solide et à former une solution secondaire de concentré.
des moyens de mélange (44) servant à mélanger la solution secondaire de concentré à la solution de concentré extraite des moyens de recyclage, afin de former ladite solution médicale.

18. Système suivant la revendication 17, caractérisé en ce qu'il comprend en outre :
un récipient tertiaire (32) contenant une substance solide soluble dans l'eau,
des moyens tertiaires de fourniture d'eau (4) servant à fournir de l'eau au récipient tertiaire afin de dissoudre la substance solide et de former une solution tertiaire de concentré,
des moyens de mélange (41) servant a mélanger la solution tertiaire de concentré à la solution de concentré extraite des moyens de recyclage, éventuellement mélangée à la solution secondaire de concentré, afin de former ladite solution médicale.

19. Système suivant l'une quelconque des revendications précédentes, caractérisé par une tuyauterie (3, 17) servant à fournir directement de l'eau provenant des moyens de fourniture d'eau (2) à l'un des récipients (31, 27, 28, 29) afin de chasser de ce dernier une quantité préfixée de la substance solide ou de la solution de concentré à base de liquide pour l'envoyer directement au réservoir (21).
